# FASCICULE DE BREVET EUROPEEN

(11) **EP 2 317 312 B1**
(45) Date de publication et mention de la délivrance du brevet: **23.09.2015**
(21) Numéro de dépôt: 10354054.8
(22) Date de dépôt: 28.09.2010
(51) Int. Cl.: G01N 33/487, G01N 27/07, G01N 27/404, A61B 5/00, G01N 27/08, G01N 27/49, A61B 10/00

(54) **Procédé et cellule de mesure de la concentration globale en ions d'un fluide corporel**
Verfahren und Zelle zum Messen der Gesamtionenkonzentration in einer Körperflüssigkeit
Method and cell for measuring the overall concentration of ions in a bodily fluid

(30) Priorité: 02.10.2009 FR 0904709
(43) Date de publication de la demande: 04.05.2011
(73) Titulaire: Commissariat à l'Énergie Atomique et aux Énergies Alternatives, 75015 Paris (FR)
(72) Inventeur: Revol-Cavalier, 38180 Seyssins (FR)
(74) Mandataire: Dubreu, Sandrine

(56) Documents cités:
- EP-A1- 1 742 063
- EP-A1- 1 878 693
- JP-A- 63 195 559
- JP-A- 2003 050 228
- US-A- 3 936 729
- US-A1- 2007 086 927
- US-A1- 2009 201 035
- US-B2- 7 351 375
- SHAMSUDDIN A K M ET AL: "Continuous monitoring of sweating by electrical conductivity measurement", PHYSIOLOGICAL MEASUREMENT, INSTITUTE OF PHYSICS PUBLISHING, BRISTOL, GB, vol. 19, no. 3, 1 août 1998 (1998-08-01), pages 375-382, XP020073849, ISSN: 0967-3334, DOI: 10.1088/0967-3334/19/3/006
- MAHABADI K A ET AL: "Restrictive dual capacitively coupled contactless conductivity detection for microchip electrophoresis", PROCEDIA CHEMISTRY, ELSEVIER, vol. 1, no. 1, 1 septembre 2009 (2009-09-01), pages 1351-1354, XP026799804, ISSN: 1876-6196, DOI: 10.1016/j.proche.2009.07.337

## Description

### Domaine technique de l'invention

L'invention concerne un procédé de mesure de concentration globale en ions d'un fluide corporel et une cellule de mesure de concentration globale en ions d'un fluide corporel.

### État de la technique

Les mesures de conductimètre sont couramment utilisées pour déterminer la concentration globale des ions contenus dans un liquide. Cette technique trouve de nombreuses applications dans le domaine de la chimie et de la biochimie pour doser et suivre l'évolution dans le temps de la concentration en ions d'une solution. La conductimétrie est utilisée, par exemple, dans le domaine environnemental, pour le suivi de la pollution des eaux des fleuves et des rivières ou encore dans le domaine médical, pour l'analyse de solutions physiologiques et/ou de fluides corporels comme la sueur d'un individu.

Les mesures de conductimétrie sont, classiquement, réalisées dans une cellule de conductimétrie comportant au moins deux électrodes en vis-à-vis, trempées dans le liquide à analyser constituant l'électrolyte. Une tension est appliquée entre les deux électrodes, généralement en platine et/ou platine platiné. Les ions présents dans le liquide à analyser ferment alors le circuit électrique et l'on mesure alors un courant d'intensité qui varie en fonction de la conductance du liquide à analyser. Ainsi, une fois étalonnée, la cellule permet de mesurer la conductivité du liquide. La conductivité est proportionnelle à la concentration ionique globale de la solution, pour des solutions fortement dissociées de type chlorure de sodium ou de potassium.

Il est connu de pratiquer des mesures de conductivité de la sueur pour diagnostiquer la mucoviscidose chez le nourrisson, à partir de la concentration en chlore sudoral. La conductivité de la sueur est mesurée puis convertie en molarité de NaCl.

Par ailleurs, la variation de la conductivité ionique ou de la résistance électrique de la sueur, fonction de la concentration des ions, apporte des informations sur les modifications physiologiques d'un individu. Ainsi, la déshydratation d'un sportif ou d'un militaire peut être suivie à partir de la variation de la conductivité ionique de la sueur.

Il existe actuellement des dispositifs de mesure utilisables pour un fluide corporel collecté au préalable sur l'individu. Pour de la sueur, cette dernière est collectée, par exemple, grâce à des membranes absorbantes en forme de patch puis analysée ultérieurement en laboratoire ou par l'individu lui-même. On peut, par exemple, estimer la prise de stupéfiants à partir de tels dispositifs.

Il existe également des dispositifs de mesure embarqués, disposés sur l'individu lui-même qui permettent une mesure directement sur l'individu, en temps réel et en continu.

À titre d'exemple, le document US-B-6198953 décrit un dispositif portatif et un procédé pour la collecte et l'analyse en continu de la sueur. Le procédé comporte la pénétration dans la peau d'une substance sudorifique, la pilocarpine, par iontophorèse afin de stimuler la sécrétion de sueur des glandes sudoripares de l'épiderme, suivie de la mesure de la conductivité électrique de la sueur collectée à partir de deux électrodes. Un circuit d'analyse teste la conductivité du flux de sueur passant entre les deux électrodes. Ces électrodes produisent alors un signal électrique proportionnel à la concentration en ions, en réponse au courant passant via la sueur.

Néanmoins, la mesure de conductimétrie impose d'appliquer une tension peu élevée, en général d'un volt, entre les électrodes et un balayage en tension. En effet, sous une tension continue élevée, les électrodes sont le siège de réactions électrochimiques et se polarisent. Les anions et des cations présents dans la solution ionique migrent alors vers les électrodes polarisées et induisent un champ électrique opposé au sens du champ imposé par le générateur de tension. Ce champ électrique, antagoniste, modifie la résistance électrique de la solution ionique et induit l'apparition de capacités parasites, sources d'erreur sur la mesure de la concentration globale en ions. Pour résoudre ce problème, une tension alternative doit être appliquée, avec un balayage en fréquence de tension. Le balayage en fréquence permet d'alterner le sens de la tension pour que le phénomène de migration ne se manifeste pas et d'inhiber les réactions électrochimiques au voisinage des électrodes. La fréquence de balayage doit alors être suffisamment élevée pour éviter ces phénomènes parasitaires. Cette fréquence liée à la nature de l'électrolyte se situe classiquement entre 10 Hertz et 1000 Hertz. La mesure de concentration ionique par conductimétrie nécessite, par conséquent, le pilotage en fréquence de la tension et la mise en place d'un circuit d'analyse adapté.

Plusieurs dispositifs utilisant un courant alternatif ont été proposés pour mesurer la conductivité. À titre d'exemple, on peut citer la publication de Mahabadi K. A. et al., "Restrictive dual capacitively coupled contactless conductivity détection for microchip electrophoresis", Procedia Chemistry, Elsevier, vol.1, no. 1, 01/09/2009, 1351-1354, la publication de Shamsuddin A. K. et al., "Continuous monitoring of sweating by electrical conductivity measurement", Physiological measurement, Institute of Physics Publishing, Bristol, GB vo1.19, no. 3, 01/08/1998, 375-382 et les documents US-A-20070086927 et US-A-20090201035.

Par ailleurs, la conductivité d'une solution ionique dépend de la géométrie de la cellule de conductimétrie. Elle est proportionnelle à la surface des électrodes de la cellule de conductimétrie et inversement proportionnelle à la distance entre les deux électrodes. Le volume de la cellule de conductimétrie doit donc être suffisamment important pour pouvoir agencer les électrodes en vis-à-vis. De façon connue, le volume minimum d'une cellule de conductimétrie ne doit pas être inférieur à 10ml pour ne pas affecter la qualité des mesures. Ces contraintes de taille constituent un frein pour la miniaturisation des dispositifs de mesure et l'utilisation de telles cellules de conductimétrie dans les systèmes embarqués sur un individu où la taille des systèmes de mesure constitue un élément critique.

Les dispositifs de mesure de conductivité de l'art antérieur utilisent des électrodes, généralement, en platine platiné, pour accroître leur surface développée. L'utilisation d'un tel matériau nécessite de fréquents étalonnages du fait de l'évolution de la surface développée par les électrodes, par exemple due à la perte de matériau. De plus, pour éviter une usure importante, de telles électrodes doivent être stockées en milieu humide.

### Objet de l'invention

L'invention a pour but de proposer un procédé de mesure de la concentration globale en ions d'un fluide corporel et une cellule de mesure remédiant aux inconvénients de l'art antérieur.

En particulier, l'invention a pour but de proposer un procédé et une cellule de mesure permettant de réaliser une mesure quantitative simple et précise de la concentration globale en ions d'un fluide corporel, en particulier de la sueur.

L'invention a également pour but de proposer une cellule de mesure pouvant être miniaturisée, pour un encombrement et un poids minimum aux fins d'être aisément intégrée dans un système de mesure embarqué sur un individu et de permettre le suivi en continu et le contrôle de l'état physiologique d'un individu.

L'invention à également pour but de proposer un procédé de mesure de la concentration globale en ions d'un fluide corporel utilisant une telle cellule de mesure.

Selon l'invention, ce but est atteint par un procédé de mesure et une cellule de mesure de la concentration en ions d'un fluide corporel selon les revendications indexées.

L'invention fournit un procédé de mesure de la concentration globale en ions d'un fluide corporel, caractérisé en ce qu'il comporte les étapes suivantes:
- application d'une tension continue et stable entre des première et seconde électrodes (5, 6) d'une cellule de mesure de la concentration globale en ions d'un fluide corporel de manière à provoquer l'apparition de réactions électrochimiques d'hydrolyse de l'eau du fluide corporel, au niveau desdites première et seconde électrodes (5, 6),
- mesure d'un courant d'hydrolyse entre les deux électrodes (5, 6), ledit courant d'hydrolyse étant engendré par lesdites réactions électrochimiques d'hydrolyse de l'eau et,
- détermination de la concentration globale ionique du fluide corporel par comparaison avec une courbe d'étalonnage définie au préalable,
en ce que ladite cellule de mesure comporte un canal fluidique (1) aménageant au moins un passage (4) permettant l'écoulement du fluide corporel d'une entrée (2) vers une sortie (3), ledit canal fluidique (1) ayant, entre les première et seconde électrodes (5, 6) disposées sur ledit passage (4), une section interne inférieure ou égale à 1,5 mm de manière à ce que la concentration ionique du fluide corporel est le facteur limitant la cinétique desdites réactions électrochimiques d'hydrolyse de l'eau,
et en ce que la cellule de mesure comporte un générateur de tension (7) et un circuit électronique de mesure (8), connectés en série aux première et seconde électrodes (5, 6).

Un autre objet de l'invention est une cellule de mesure de concentration globale en ions d'un fluide corporel qui comporte :
- un canal fluidique (1) avec une entrée (2) et une sortie (3) du fluide corporel, ledit canal fluidique (1) comportant des première et seconde zones (Z₁, Z₂) ayant chacune une section interne déterminée et une troisième zone (Z₃) située entre la première zone (Z₁) et la seconde zone (Z₂) et adjacente auxdites première et seconde zones (Z₁, Z₂) et aménageant au moins un passage (4) pour le fluide corporel, ladite troisième zone (Z₃) ayant une section interne réduite, inférieure aux sections internes respectives desdites première et seconde zones (Z₁, Z₂), pour limiter le transport ionique entre des première et seconde électrodes (5, 6) dans le fluide corporel, la section interne de la troisième zone (Z₃) étant inférieure ou égale à 1,5 mm, lesdites première et seconde électrodes (5, 6) étant disposées sur ledit passage (4) et situées, respectivement, au niveau des première et seconde zones (Z₁, Z₂),
- un générateur de tension (7) et un circuit électronique de mesure (8), connectés en série auxdites première et seconde électrodes (5, 6),
le générateur de tension (8) étant un générateur de tension continue et stable,
ledit générateur étant configuré pour appliquer une différence de potentiel entre les deux électrodes (5, 6) pour générer l'hydrolyse de l'eau du fluide corporel au niveau desdites première et seconde électrodes (5, 6), le circuit électronique de mesure étant configuré pour mesurer le courant d'hydrolyse entre les deux électrodes (5, 6).

### Description sommaire des dessins

D'autres avantages et caractéristiques ressortiront plus clairement de la description qui va suivre des modes particuliers de réalisation de l'invention donnés à titre d'exemples non limitatifs et représentés aux dessins annexés, dans lesquels :
- La figure 1 représente, schématiquement et en coupe, un mode particulier de réalisation d'une cellule de mesure de concentration en ions d'un fluide corporel selon l'invention.
- La figure 2 représente une cellule de mesure, schématiquement et en coupe selon l'axe AA de la figure 1.
- Les figures 3 et 5 représentent, schématiquement et en coupe, des variantes de la figure 1.
- Les figures 4 et 6 représentent une cellule de mesure, schématiquement et en coupe, respectivement, selon l'axe BB de la figure 3 et CC de la figure 5.
- La figure 7 représente, schématiquement et en perspective, un mode particulier de réalisation d'une cellule de mesure selon l'invention.
- La figure 8 représente une cellule de mesure, schématiquement et en coupe, selon l'axe DD de la figure 7.
- La figure 9 représente, schématiquement et en coupe, une variante de la figure 8.
- Les figures 10 et 12 représentent, schématiquement et en perspective, des variantes de la figure 7.
- Les figures 11 et 13 représentent, schématiquement, une cellule de mesure en coupe, respectivement, selon l'axe EE de la figure 10 et FF de la figure 12.
- La figure 14 représente, schématiquement, un mode de réalisation particulier d'une cellule de mesure selon l'invention.
- Les figures 15 et 18 représentent le courant d'hydrolyse, en fonction du temps, mesuré à partir d'une cellule de mesure selon l'invention et de solutions étalon.
- La figure 16 correspond à une courbe d'étalonnage représentant la valeur moyenne du courant d'hydrolyse collecté en fonction de la concentration, obtenue à partir de la figure 15.
- Les figures 17 et 20 représentent une courbe d'étalonnage obtenue à partir, respectivement, du graphe de la figure 15 et de la figure 19, avec le report de la valeur moyenne du courant d'hydrolyse collecté d'un échantillon de sueur.
- La figure 19 représente la valeur moyenne du courant d'hydrolyse en fonction du temps, obtenue à partir du graphe de la figure 18.

### Description des modes de réalisation particuliers de l'invention

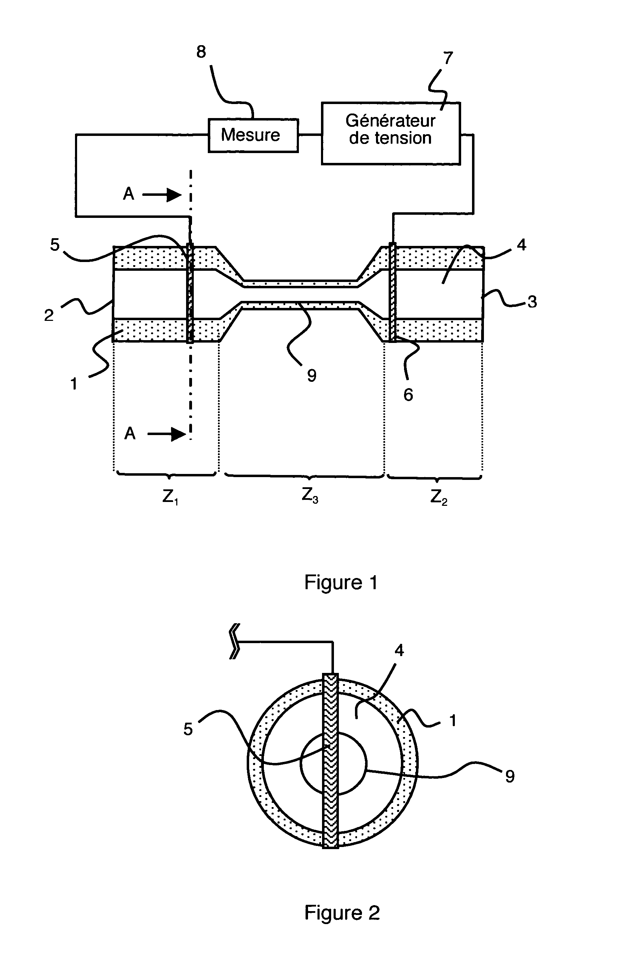

Comme représenté à la figure 1, une cellule de mesure de concentration globale en ions d'un fluide corporel comporte un canal fluidique 1 avec une entrée 2 et une sortie 3 du fluide corporel. Le canal fluidique 1 aménage au moins un passage 4 pour le fluide corporel, permettant l'écoulement du fluide corporel de l'entrée 2 vers la sortie 3.

La cellule de mesure comporte également des première et seconde électrodes, respectivement 5 et 6, connectées à un générateur de tension 7 et à un circuit électronique de mesure 8. Le générateur de tension 7 et le circuit électronique de mesure 8 sont classiquement montés en série.

Le générateur de tension 7 peut, avantageusement, être un potentiostat ou une pile, par exemple de 4,5V.

Le générateur de tension 7 et le circuit électronique de mesure 8 sont montés en série afin de permettre la mesure d'un courant électrique. Le circuit électronique de mesure 8 peut, avantageusement, être un ampèremètre. Alternativement, le circuit électronique de mesure 8 peut être un dispositif de mesure permettant la détermination du flux d'électrons collecté par les électrodes, respectivement 5 et 6. De façon préférée, ce dispositif de mesure 8 est relié à un système d'exploitation de données, ce dernier pouvant être intégré au circuit électronique de mesure 8, sous la forme d'une carte électronique ou d'un ASIC ("Application Specific Integrated Component").

Le canal fluidique 1 a, entre les première et seconde électrodes, respectivement 5 et 6, une section interne inférieure ou égale à 1,5 mm, pour limiter le transport ionique entre lesdites électrodes, 5 et 6. Avantageusement, la cellule de mesure comporte un rétrécissement 9 de la section interne du canal fluidique, entre les première et seconde électrodes, respectivement 5 et 6.

Le canal fluidique 1 peut être un capillaire ayant une section interne continue. La limitation du transport ionique entre lesdites électrodes, 5 et 6, est induite par la faible section interne du capillaire, inférieure ou égale à environ 1,5 mm.

Avantageusement, la cellule de mesure comporte un rétrécissement 9 de la section interne du canal fluidique, entre les première et seconde électrodes, respectivement 5 et 6. Le canal fluidique 1 peut avoir une section interne plus grande que 1,5 mm, et au moins un rétrécissement 9 localisé entre les première et seconde électrodes, respectivement 5 et 6, diminuant la section interne jusqu'à une valeur inférieure ou égale à 1,5 mm. Le ou les rétrécissements 9 sont alors responsables de la limitation du transport ionique. Ce mode de réalisation est préféré car cette cellule de mesure est simple à fabriquer et évite l'utilisation de petites électrodes coûteuses.

Selon un mode de réalisation particulier, le canal fluidique 1 comporte des première et seconde zones, respectivement Z₁ et Z₂, ayant chacune une section interne déterminée, et une troisième zone Z₃ située entre la première zone Z₁ et la seconde zone Z₂, et adjacente auxdites première et seconde zones Z₁ et Z₂. La première zone Z₁ est reliée à l'entrée 2 et la seconde zone Z₂ à la sortie 3. Les trois zones, respectivement Z₁, Z₂ et Z₃, sont contiguës de sorte que l'écoulement du fluide corporel dans le passage 4 peut s'effectuer de l'entrée 2 vers la sortie 3.

La troisième zone Z₃ a une section interne réduite, inférieure aux sections internes respectives des première et seconde zones, Z₁ et Z₂, pour limiter le transport ionique, dans le fluide corporel, entre les première et seconde électrodes, respectivement 5 et 6. On entend par section interne d'une zone, la surface d'intersection entre le canal fluidique 1 et un plan perpendiculaire à la direction d'écoulement du fluide, sur la zone concernée. La surface d'intersection prise en considération est celle délimitée par la paroi interne du canal fluidique 1. Lorsque la section interne varie le long de la zone concernée, la valeur prise en compte pour comparer les zones Z₁, Z₂ et Z₃ est la valeur de section interne la plus petite pour chaque zone concernée.

La première électrode 5 et la seconde électrode 6 sont disposées sur le passage 4 du canal fluidique 1 et situées, respectivement, au niveau des première et seconde zones, respectivement Z₁ et Z₂. Ainsi, les première et seconde électrodes, respectivement 5 et 6, sont disposées sur le passage 4 du fluide corporel de sorte qu'au moins une partie de chaque électrode 5 et 6 soit en contact avec le fluide corporel.

Les première et seconde électrodes, respectivement 5 et 6, peuvent être soit une électrode de travail et une électrode de référence soit une électrode de travail et une contre-électrode.

Selon un mode de réalisation particulier représenté aux figures 1 et 2, le canal fluidique 1 peut avoir une forme tubulaire. À titre d'exemple, le canal fluidique 1 peut être un tube souple ou rigide en matériau inerte, de préférence en matériau plastique. Le diamètre intérieur du tube au niveau du rétrécissement 9 peut, avantageusement, varier entre 0,1mm et 1,5mm. En dessous de 0,1 mm, le courant mesuré par les électrodes, 5 et 6, est généralement trop faible, du fait d'un transport ionique insuffisant à travers le rétrécissement 9. Au-dessus de 1,5 mm, le courant d'hydrolyse génère généralement trop de bulles au niveau des électrodes, 5 et 6. Les bulles perturbent alors les mesures et sont source d'erreurs.

La première zone Z₁ débouche directement sur l'entrée 2 et la seconde zone Z₂ sur la sortie 3. Le canal fluidique 1 peut comporter au moins un rétrécissement 9 du tube au niveau de la troisième zone Z₃. Le rétrécissement 9 forme un goulot d'étranglement du passage 4 qui rend le passage 4 plus étroit au niveau de la troisième zone Z₃.

Les première et seconde électrodes, respectivement 5 et 6, peuvent être des électrodes à base de carbone ou métalliques, par exemple en acier, or, aluminium, cuivre, étain ou platine. De préférence, les électrodes 5 et 6 sont des électrodes en platine.

Comme représenté à la figure 2, les première et seconde électrodes, respectivement 5 et 6, sont constituées chacune par un fil traversant le canal fluidique 1 et disposées face à face. Les première et seconde électrodes, respectivement 5 et 6, sont par exemple des fils de platine ayant un diamètre compris entre 0,2 mm et 1 mm et espacés l'un de l'autre, dans le sens de l'écoulement du fluide (de gauche à droite à la figure 1) d'une distance variant de quelques millimètres à quelques centimètres, avantageusement, d'une distance comprise entre 2mm et 20mm.

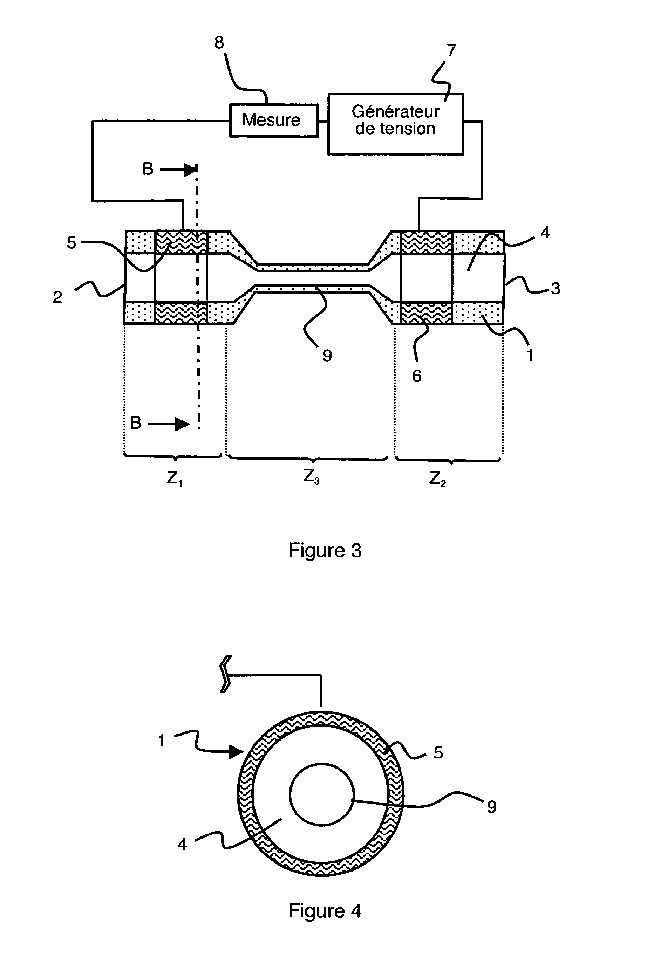

Selon une variante représentée aux figures 3 et 4, le canal fluidique 1 est un conduit annulaire et les première et seconde électrodes, respectivement 5 et 6, sont également annulaires et formées dans la paroi annulaire du canal fluidique 1. Avantageusement, les première et seconde électrodes, respectivement 5 et 6, sont en platine. Alternativement, seule la surface des première et seconde électrodes, respectivement 5 et 6, destinée à être en contact avec le fluide corporel, est recouverte d'une couche de platine. Le dépôt de la couche de platine peut être réalisé par des techniques classiques d'évaporation, de pulvérisation ou de dépôt électrochimique.

À titre d'exemple, le diamètre du conduit annulaire constituant les première et seconde électrodes, respectivement 5 et 6, peut être compris entre 0,1mm et 10mm et la distance entre les deux électrodes 5 et 6, entre 1 mm et 200mm.

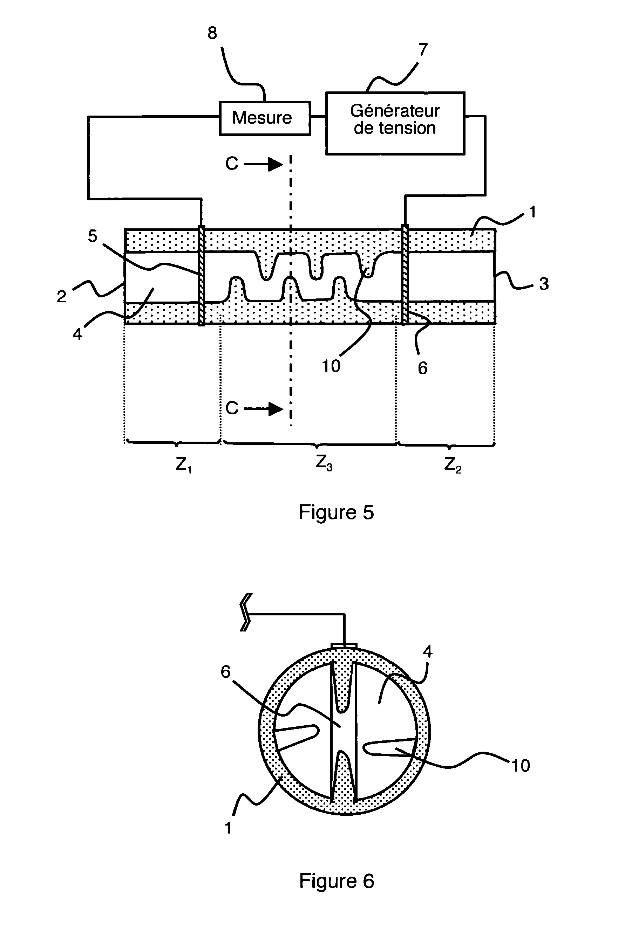

Selon une autre variante représentée aux figures 5 et 6, le canal fluidique 1 peut présenter, au niveau de la troisième zone Z₃, des protubérances 10 à la surface de la paroi interne du canal fluidique 1, à la place du rétrécissement 9. Les protubérances 10 réduisent la section interne du canal fluidique 1 au niveau de la troisième zone Z₃. Ainsi, les protubérances 10 entravent l'écoulement du fluide corporel et freinent la circulation du fluide corporel entre les première et seconde électrodes, respectivement 5 et 6. L'importance de la limitation du transport ionique entre les deux électrodes, respectivement 5 et 6, dans le fluide corporel, dépendra du nombre de protubérances 10 présentes dans la troisième zone Z₃ et de la section interne de cette troisième zone Z₃.

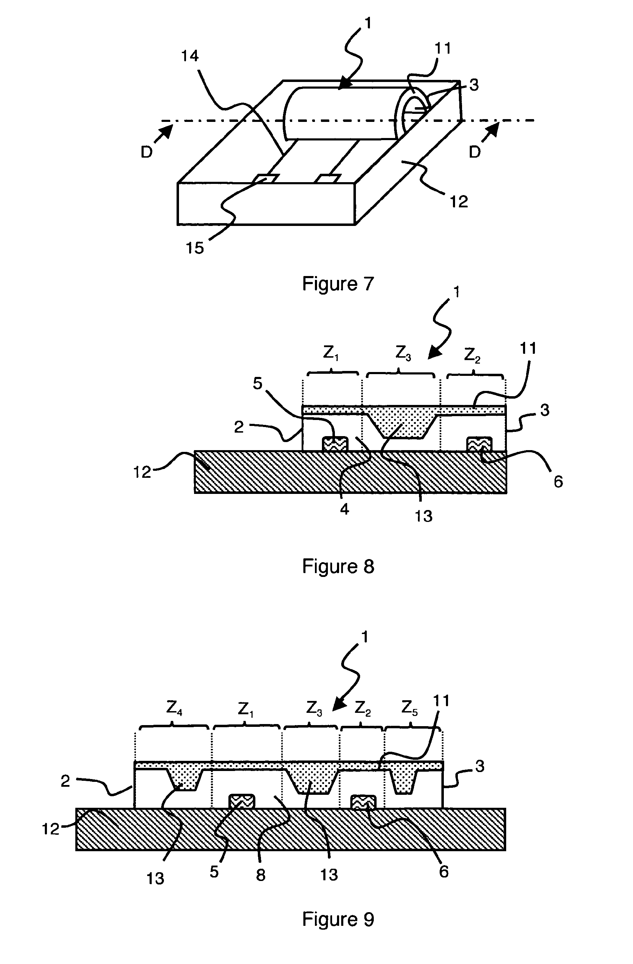

Selon un autre mode de réalisation particulier représenté à la figure 7, le canal fluidique 1 de la cellule de mesure est délimité par un capot 11 et par au moins une partie d'un support 12, le capot 11 étant disposé sur le support 12. Le canal fluidique 1 est constitué par le capot 11 en partie haute (en haut à la figure 8) et par au moins une partie d'une face plane du support 12 en partie basse (en bas à la figure 8). Le canal fluidique 1 a, par exemple, une forme hémicylindrique. Le passage 4 est, par conséquent, délimité par la paroi interne du capot 11 et la surface du support 12 en vis-à-vis du capot 11.

La paroi du capot 11 comporte, avantageusement, une épaisseur plus importante formant saillie 13 sur le passage 4. La saillie 13 forme une section interne réduite et circonscrit alors la troisième zone Z₃.

Le support 12 peut être constitué par un matériau souple, de préférence choisi parmi les matériaux polymères, les matériaux textiles à base de fibres synthétiques ou végétales, naturelles ou transformées, ou les matériaux à base de fibres cellulosiques comme le papier.

Selon une autre variante, le support 12 peut être un matériau rigide ou semi-rigide, de préférence choisi parmi le silicium et le verre.

Comme représenté à la figure 8, les première et seconde électrodes, respectivement 5 et 6, sont déposées sur une même paroi du canal fluidique 1, avantageusement, sur une même paroi du support 12. Les première et seconde électrodes, respectivement 5 et 6, peuvent être déposées sur le support 12, par exemple sous forme de pastilles, par des techniques conventionnelles de dépôt utilisées pour les circuits imprimés ou circuits intégrés. Les première et seconde électrodes, respectivement 5 et 6, ainsi formées sont coplanaires c'est-à-dire disposées côte à côte l'une par rapport à l'autre dans le sens longitudinal du passage 4 (gauche à droite à la figure 8).

Les première et seconde électrodes, respectivement 5 et 6, sont de préférence reliées au générateur de tension 7 et au circuit électronique de mesure 8 par des moyens de connexion intégrés dans le support 12. Les moyens de connexion comportent classiquement des fils de connexion 14 et un système de raccordement électrique 15. Le système de raccordement électrique 15 est, de préférence, disposé à l'extrémité du support 12 et destiné à être connecté au générateur de tension 7 et au circuit électronique de mesure 8 de la cellule de mesure.

Selon une variante représentée à la figure 9, la cellule de mesure comporte une ou deux zones supplémentaires Z₄ et/ou Z₅. Plus particulièrement, la cellule de mesure comporte une zone supplémentaire Z₄ adjacente à la première zone Z₁ et située entre l'entrée 2 et la première zone Z₁ et une zone supplémentaire Z₅ adjacente à la seconde zone Z₂ et située entre la seconde zone Z₂ et la sortie 3. Chaque zone supplémentaire Z₄ et Z₅ a une section interne réduite, inférieure aux sections internes respectives des première et seconde zones, respectivement Z₁ et Z₂. Les tronçons de canal fluidique 1, correspondant respectivement aux zones supplémentaires Z₄ et Z₅, peuvent être identiques ou différents. La présence d'une zone supplémentaire Z₄ et/ou Z₅ permet, en particulier, de réguler le flux du fluide corporel pour des cellules de mesure destinées à être immergées dans le fluide corporel.

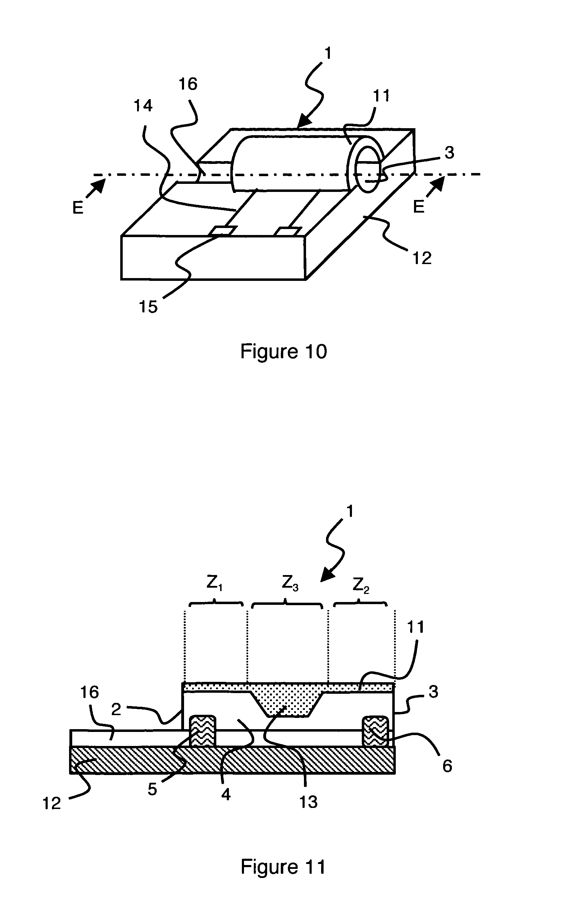

Selon une variante, le support 12 peut également comporter des moyens de drainage du fluide corporel vers le canal fluidique 1. Comme représenté aux figures 10 et 11, les moyens de drainage sont constitués par au moins une rainure 16 dans le support 12. La rainure 16 peut, par exemple, être réalisée en continu, d'un bout à l'autre du support 12 (gauche à droite aux figures 10 et 11). La rainure 16 est recouverte, sur une partie de sa longueur, par le capot 11. Le capot 11 est également disposé en vis-à-vis de la rainure 16 (figure 10). Les première et seconde électrodes, respectivement 5 et 6, sont déposées au fond de la rainure 16, respectivement dans la première zone Z₁ et dans le seconde zone Z₂ et connectées comme décrit ci-dessus au générateur de tension 7 et au circuit électronique de mesure 8. Des moyens de drainage peuvent également être reliés à la sortie 3 du canal fluidique 1 afin de faciliter l'évacuation de la sueur au cours des mesures. Ces moyens de drainage peuvent être une seringue mécanique, une pompe fluidique passive, constituée par exemple d'un canal en tissu hydrophile bordé d'un canal en tissu hydrophobe, débouchant sur un absorbeur hydrophile (non représenté).

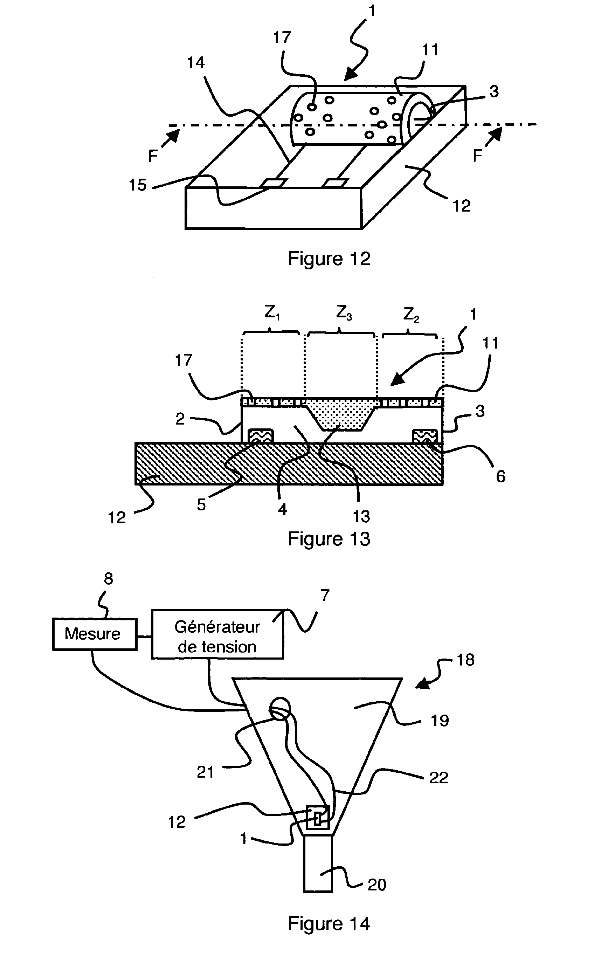

Selon une autre variante représentée aux figures 12 et 13, le capot 11 peut comporter au moins un orifice de vidange 17 traversant la paroi du capot 11. L'orifice de vidange 17 est situé en dehors de la troisième zone Z₃ afin de ne pas diminuer l'effet de limitation de la circulation des ions entre les première et seconde électrodes, respectivement 5 et 6. Avantageusement, le capot 11 comporte plusieurs orifices de vidange 17. Le ou les orifices de vidange 17 sont destinés à favoriser l'évacuation du fluide corporel. Cette variante est particulièrement adaptée à une cellule de mesure destinée à être immergée dans le fluide corporel.

Selon un mode de réalisation particulier, la cellule de mesure comporte, avantageusement, un dispositif de collecte de fluide corporel qui alimente le canal fluidique 1 en fluide corporel. Le dispositif de collecte peut être un système embarqué, par exemple, une poche à sueur disposée directement sur un individu. La poche à sueur permet de recueillir en temps réel la sueur d'un individu et la canalise vers l'entrée 2 du canal fluidique 1. La sueur s'écoule dans le passage 4 puis est évacuée par la sortie 3. La cellule de mesure selon ce mode de réalisation est particulièrement adaptée pour le suivi en temps réel des variations de concentration en ions, par exemple, de la sueur d'un individu. Le dispositif de collecte peut être connecté directement à l'entrée 2 du canal fluidique 1 par un conduit (non représenté) ou relié au support 12.

Selon un mode de réalisation particulier représenté à la figure 14, le support 12 du canal fluidique 1 forme au moins une partie d'un dispositif de collecte 18 constitué, par exemple, par une poche à sueur. Le dispositif de collecte 18 a une forme d'entonnoir avec une partie évasée 19 (en haut à la figure 14) et une partie étroite 20 (en bas à la figure 14). La partie évasée 19 sert de réceptacle pour recueillir la transpiration émise par la peau et la partie étroite 20 permet d'évacuer la sueur. Le support 12 peut, avantageusement, être intégré dans la paroi du dispositif de collecte 18, de préférence, au niveau de la partie inférieure évasée 19 ou de la partie supérieure étroite 20 de sorte que le canal fluidique 1 puisse être immergé dans la sueur collectée.

Selon une variante non représentée, le support 12 peut être collé ou déposé sur la paroi interne du dispositif de collecte 18.

Comme représenté à la figure 14, une ouverture 21 peut être réalisée, par exemple, dans la partie supérieure évasée 19, pour faire passer des fils de circuit électrique 22 reliant le générateur de tension 7 et le circuit électronique de mesure 8 au système de branchement 15, à travers la paroi du dispositif de collecte 18.

Selon un mode particulier de réalisation, un procédé de mesure de la concentration globale en ions d'un fluide corporel au moyen de la cellule de mesure décrite ci-dessus comporte l'application d'une tension continue et stable entre les première et seconde électrodes, respectivement 5 et 6. La tension appliquée est comprise, avantageusement, entre 2V et 10V, de préférence, entre 3V et 5V. La tension est, par exemple, appliquée durant une durée comprise entre 0,1 s à 10s, de préférence, entre 0,1 s et 4s.

Le fluide corporel collecté est canalisé vers le canal fluidique 1 de la cellule de mesure, par exemple par un dispositif de collecte 18, et remplit au moins partiellement le passage 4. La présence du fluide corporel entre les première et seconde électrodes, respectivement 5 et 6, permet alors de réaliser une mesure électrochimique, le fluide corporel jouant alors le rôle d'un électrolyte.

Le générateur de tension 7 et le circuit électronique de mesure 8 peuvent, également, être intégrés au dispositif de collecte 18 sous la forme d'un circuit intégré souple, relié aux électrodes 5 ou 6. Selon une variante, les électrodes, 5 et 6, sont intégrées au circuit intégré souple. Le circuit intégré souple est alors configuré de manière à ce que les électrodes 5 et 6 soient en contact avec le liquide contenu dans le dispositif de collecte 18.

L'application d'une tension continue et stable entre les première et seconde électrodes, respectivement 5 et 6, provoque l'apparition de réactions électrochimiques d'hydrolyse de l'eau du fluide corporel, au niveau des première et seconde électrodes, respectivement 5 et 6. En effet, lorsqu'on applique une tension continue et stable entre les première et seconde électrodes, respectivement 5 et 6, les électrodes 5 et 6 sont le siège des réactions électrochimiques prépondérantes suivantes :
A l'anode

   H₂O → ½ O₂ + 2H⁺ + 2é
A la cathode

   2H₂O + 2é → H₂ + 2OH⁻

Le courant engendré correspond à un courant d'hydrolyse de l'eau du fluide corporel. Ce courant d'hydrolyse dépend de plusieurs paramètres, notamment la surface des électrodes, la température, la tension appliquée, la concentration et les coefficients de diffusion des espèces entrant en jeu dans les réactions électrochimiques.

L'accumulation des ions OH⁻ et H⁺, respectivement à la cathode et à l'anode, créée un courant d'hydrolyse qui dépend de la cinétique des réactions d'hydrolyse se déroulant aux première et seconde électrodes, respectivement 5 et 6. Sous l'action du champ électrique, les ions OH⁻ et H⁺ acquièrent une vitesse limite proportionnelle au champ appliqué. Les ions, cations et anions, présents dans le fluide corporel jouent le rôle de porteurs de charges et constituent des sels porteurs. Les sels porteurs véhiculent les charges entre les première et la seconde électrodes, respectivement 5 et 6, fermant ainsi le circuit. La concentration en sels porteurs, comprise généralement entre 0,005mol.l⁻¹ et 0,1mol.l⁻¹, est suffisante pour transporter les électrons assez vite pour ne pas limiter la cinétique des réactions électrochimiques qui se déroulent à l'interface entre le fluide corporel et la première ou la seconde électrode, respectivement 5 et 6.

La présence d'un canal fluidique 1 et d'une troisième zone Z₃ à section réduite dans la cellule de mesure permet de contrôler la circulation du fluide corporel et de limiter le déplacement des ions présents dans le fluide corporel, entre les première et second électrodes, respectivement 5 et 6. La concentration ionique du fluide corporel est alors le facteur limitant la cinétique des réactions d'hydrolyse. Le courant d'hydrolyse mesuré est, par conséquent, proportionnel à la concentration ionique du fluide corporel.

Le procédé comporte la mesure du courant d'hydrolyse engendré par les réactions électrochimiques d'hydrolyse de l'eau. La lecture du courant d'hydrolyse induit par l'application de la tension entre les première et seconde électrodes, respectivement 5 et 6, permet de déterminer la concentration équivalente en ions d'un fluide corporel. On mesure donc le courant d'hydrolyse, avantageusement, par chronoampérométrie, durant l'application de la tension. Les données peuvent être collectées et/ou traitées pendant ou après la mesure du courant d'hydrolyse. En particulier, une moyenne du courant d'hydrolyse est calculée à partir des données collectées sur le temps total de l'application de la tension ou sur une partie de ce temps. À titre d'exemple, une moyenne du courant d'hydrolyse est calculée à partir des valeurs de courant d'hydrolyse collectées entre la première seconde et la cinquième seconde, pour un temps d'application de la tension de 5 secondes.

On détermine la concentration globale ionique du fluide corporel par comparaison avec une courbe d'étalonnage définie au préalable.

La courbe d'étalonnage est réalisée à partir de solutions étalon ayant des concentrations en ions connues, dans des conditions identiques à celles du fluide corporel étudié. La ou les valeurs de courant d'hydrolyse obtenues pour le fluide corporel sont reportées sur la courbe d'étalonnage pour obtenir la concentration équivalente en ions du fluide corporel étudié.

Selon une variante, le procédé de mesure comporte avant application de la tension, une étape d'immersion de la cellule de mesure dans le fluide corporel. Une fois la cellule de mesure immergée dans le fluide corporel, la tension est appliquée entre les première et seconde électrodes, respectivement 5 et 6, puis les mesures de courant d'hydrolyse sont effectuées durant l'application de la tension.

### Exemple 1

Des mesures de chronoampérométrie avec un Potentiostat Mico Autolab Type III sont réalisées à partir d'une cellule de mesure, selon la figure 1, ayant un canal fluidique 1 constitué par un tube en plastique de 0,6 mm de diamètre intérieur et 1 mm extérieur, traversé par des première et seconde électrodes, 5 et 6, sous forme de fil platine de 0,6mm de diamètre, espacées l'une de l'autre de 1 cm. La première étape consiste à élaborer une courbe d'étalonnage à partir de solutions étalon de chlorure de sodium respectivement à une concentration molaire de 10 mM, 30mM, 50mM, 70mM et 100mM. Une tension est appliquée entre les première et seconde électrodes, 5 et 6, en platine.

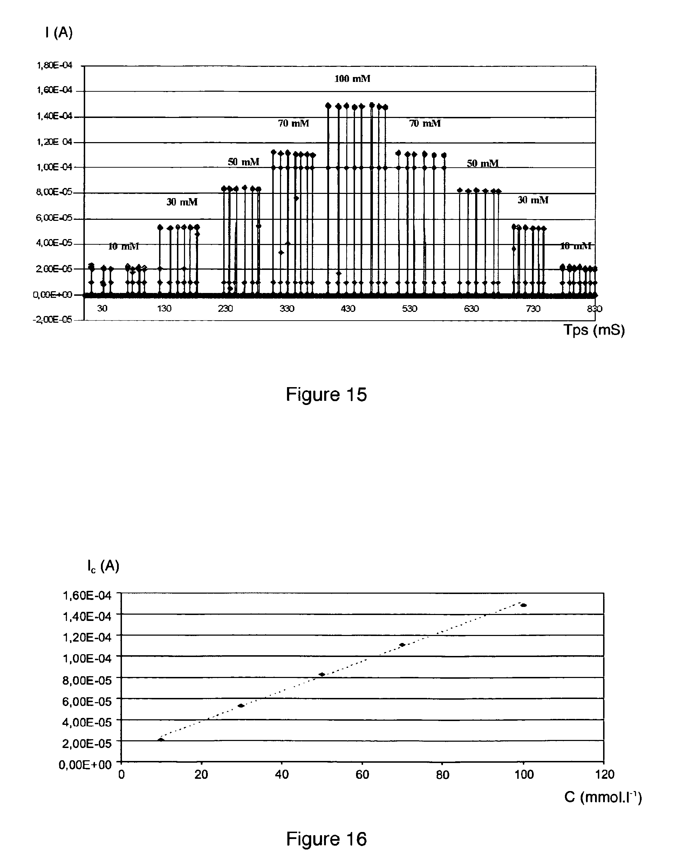

Comme représenté à la figure 15, plusieurs séries de mesures de courant d'hydrolyse sont réalisées avec un intervalle entre chaque série de mesures selon les paramètres suivants :
Durée de collecte du courant : 0,5 s
Échantillonnage : 1 mesure toutes les 100ms

Les collectes successives permettent de mesurer une valeur moyenne de courant collecté, appelée palier de courant I_{c}, et assurent une meilleure reproductibilité des mesures. Comme illustré à la figure 16, pour chaque série de mesures, le palier de courant I_{c} est déterminé et une droite est obtenue, avec un coefficient de corrélation R² de 0,997, en reportant les valeurs de palier de courant en fonction de la concentration, I_{c} =f(C).

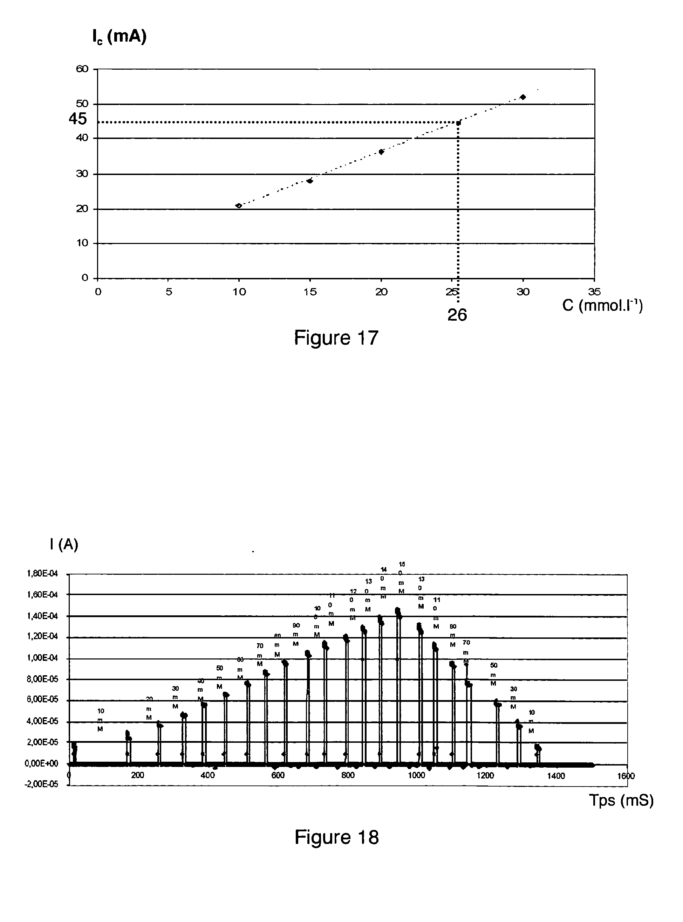

Comme illustré à la figure 17, à partir de cette courbe étalonnage, on peut déterminer une concentration globale ionique d'un échantillon de sueur collectée sur un individu, en reportant la valeur de palier de courant d'hydrolyse mesurée à partir de cet échantillon. Pour une valeur I_{c} de 45mA, on lit sur la courbe d'étalonnage la valeur correspondante de concentration globale en ions, équivalente à 26mmol.l⁻¹ de NaCl.

### Exemple 2

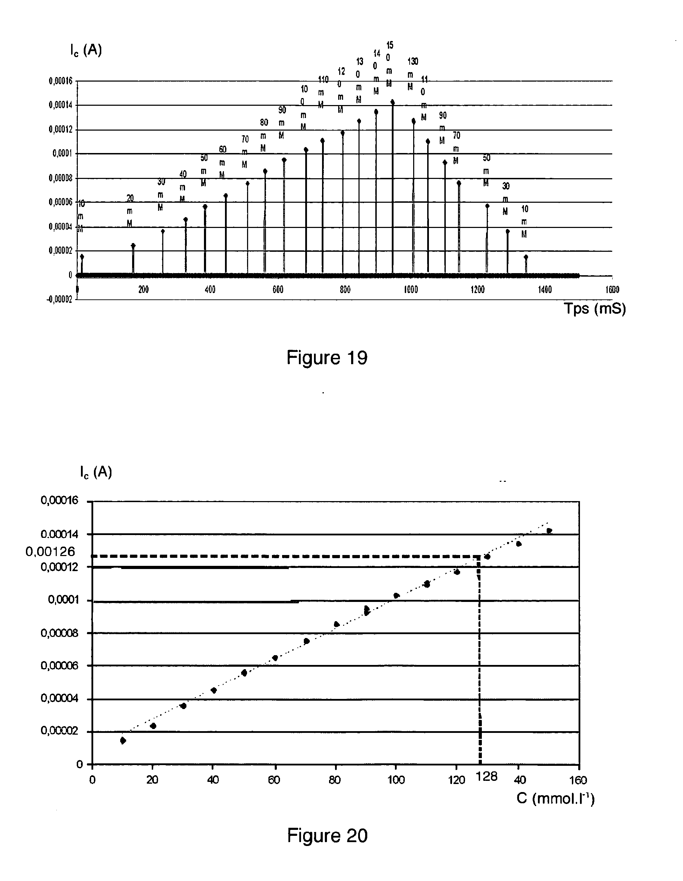

Les graphes représentés aux figures 18 et 19 sont obtenues à partir de solutions étalon de chlorure de sodium, respectivement, à une concentration molaire de 10 mM, 20mM, 30mM, 40mM, 50mM, 60mM, 70mM, 80mM, 90mM, 100mM, 110mM, 120mM, 130mM, 140mM et 150mM, dans des conditions identiques à l'exemple 1, à l'exception de la durée, plus longue, du temps de collecte du courant égale à 5 secondes au lieu de 0,5 seconde. La courbe d'étalonnage représentée à la figure 20 est réalisée à partir de la figure 19. Une droite est obtenue avec un coefficient de corrélation R² de 0,997. L'utilisation d'une durée de collecte de courant de quelques secondes permet d'éviter l'apparition de bulles au voisinage des électrodes 5 et 6. Pour un courant d'hydrolyse I_{c} de 0,00126A, la concentration globale en ions est équivalente à 128mmol.l⁻¹ de NaCl.

Le procédé est particulièrement adapté à un fluide corporel composé en majorité d'une solution aqueuse électrolytique. Le fluide corporel peut être un liquide physiologique dont la variation de concentration en ions peut indiquer des états pathologiques et, par conséquent, permettre le diagnostic de certaines maladies comme la mucoviscidose. Les fluides corporels utilisés sont, de préférence, des solutions aqueuses contenant des ions fortement dissociés comme la sueur, la salive ou l'urine et ayant une concentration en ions comprise entre 0,005 Mol.l⁻¹ et 0,1 Mol.l⁻¹. Le procédé permet, avantageusement, de mesurer la concentration globale en ions de la sueur et d'obtenir une concentration équivalente en chlorure de sodium (NaCl), sel porteur majoritaire dans la sueur.

Le procédé de mesure est simple à mettre en place, peu coûteux et permet d'obtenir rapidement des résultats précis et fiables. L'application d'une tension continue et stable présente également l'avantage de limiter la complexité de l'électronique et d'éviter le pilotage en tension.

Dans un souci de miniaturisation, des cellules de mesure peu encombrantes peuvent être réalisées en appliquant les techniques de dépôt de la microélectronique. La cellule de mesure selon l'invention peut aisément être intégrée dans un système embarqué telle qu'une poche à sueur installée sur un individu. Ainsi, le suivi et le contrôle de l'état physiologique d'un individu peuvent être réalisés en continu.

## Revendications

1. Procédé de mesure de la concentration globale en ions d'un fluide corporel, **caractérisé en ce qu'**il comporte les étapes suivantes:
- application d'une tension continue et stable entre des première et seconde électrodes (5, 6) d'une cellule de mesure de la concentration globale en ions d'un fluide corporel de manière à provoquer l'apparition de réactions électrochimiques d'hydrolyse de l'eau du fluide corporel, au niveau desdites première et seconde électrodes (5, 6),
- mesure d'un courant d'hydrolyse entre les deux électrodes (5, 6), ledit courant d'hydrolyse étant engendré par lesdites réactions électrochimiques d'hydrolyse de l'eau et,
- détermination de la concentration globale ionique du fluide corporel par comparaison avec une courbe d'étalonnage définie au préalable,
**en ce que** ladite cellule de mesure comporte un canal fluidique (1) aménageant au moins un passage (4) permettant l'écoulement du fluide corporel d'une entrée (2) vers une sortie (3), ledit canal fluidique (1) ayant, entre les première et seconde électrodes (5, 6) disposées sur ledit passage (4), une section interne inférieure ou égale à 1,5 mm
de manière à ce que la concentration ionique du fluide corporel est le facteur limitant la cinétique desdites réactions électrochimiques d'hydrolyse de l'eau,
et **en ce que** la cellule de mesure comporte un générateur de tension (7) et un circuit électronique de mesure (8), connectés en série aux première et seconde électrodes (5, 6).

2. Procédé selon la revendication 1, **caractérisé en ce que** la mesure du courant d'hydrolyse est réalisée par chronoampérométrie durant l'application de la tension continue.

3. Procédé selon l'une des revendications 1 et 2, **caractérisé en ce que** la cellule de mesure comporte un rétrécissement (9) de la section interne du canal fluidique entre les première et seconde électrodes. (5, 6).

4. Procédé selon l'une quelconque des revendications 1 à 3, **caractérisé en ce que** la tension appliquée est comprise entre 2V et 10V, de préférence entre 3V et 5V.

5. Procédé selon l'une quelconque des revendications 1 à 4, **caractérisé en ce qu'**il comporte une étape d'immersion de la cellule de mesure dans le fluide corporel, avant application de la tension.

6. Cellule de mesure de concentration globale en ions d'un fluide corporel qui comporte :
- un canal fluidique (1) avec une entrée (2) et une sortie (3) du fluide corporel,
ledit canal fluidique (1) comportant des première et seconde zones (Z₁, Z₂) ayant chacune une section interne déterminée et une troisième zone (2₃) située entre la première zone (Z₁) et la seconde zone (Z₂) et adjacente auxdites première et seconde zones (Z₁, Z₂) et aménageant au moins un passage (4) pour le fluide corporel, ladite troisième zone (Z₃) ayant une section interne réduite, inférieure aux sections internes respectives desdites première et seconde zones (Z₁, Z₂), pour limiter le transport ionique entre des première et seconde électrodes (5, 6) dans le fluide corporel, la section interne de la troisième zone (Z₃) étant inférieure ou égale à 1,5 mm,
lesdites première et seconde électrodes (5, 6) étant disposées sur ledit passage (4) et situées, respectivement, au niveau des première et seconde zones (Z₁, Z₂),
- un générateur de tension (7) et un circuit électronique de mesure (8), connectés en série auxdites première et seconde électrodes (5, 6),
le générateur de tension (8) étant un générateur de tension continue et stable, ledit générateur étant configuré pour appliquer une différence de potentiel entre les deux électrodes (5, 6) pour générer l'hydrolyse de l'eau du fluide corporel au niveau desdites première et seconde électrodes (5, 6),
le circuit électronique de mesure étant configuré pour mesurer le courant d'hydrolyse entre les deux électrodes (5, 6).

7. Cellule de mesure selon la revendication 6, **caractérisée en ce que** le cantal fluidique (1) est délimité par un capot (11) et par au moins une partie d'un support (12), ledit capot (11) étant disposé sur le support (12).

8. Cellule de mesure selon la revendication 7, **caractérisée en ce que** le support (12) comporte des moyens de drainage (16) du fluide corporel vers le canal fluidique (1).

9. Cellule de mesure selon l'une des revendications 7 et 8, **caractérisée en ce que** le capot (11) comporte au moins un orifice de vidange (17) traversant la paroi du capot (11) et situé en dehors de la troisième zone (Z₃).

10. Cellule de mesure selon l'une quelconque des revendications 7 à 9, **caractérisée en ce qu'**elle comporte un dispositif de collecte (18) de fluide corporel et **en ce que** le support (12) du canal fluidique (1) forme au moins une partie dudit dispositif de collecte (18).

11. Cellule de mesure selon l'une quelconque des revendications 6 à 10, **caractérisée en ce que** le canal fluidique (1) comporte au moins un rétrécissement (9) au niveau de la troisième zone (Z₃), rendant le passage (4) plus étroit.

12. Cellule de mesure selon l'une quelconque des revendications 6 à 11, **caractérisée en ce que** les première ou seconde électrodes (5, 6) sont constituées chacune par un fil traversant le canal fluidique (1).

13. Cellule de mesure selon l'une quelconque des revendications 6 à 11, **caractérisée en ce que** les première et seconde électrodes (5, 6) sont formées dans la paroi du canal fluidique (1).

14. Cellule de mesure selon l'une quelconque des revendications 6 à 11, **caractérisée en ce que** les première et seconde électrodes (5, 6) sont déposées sur une même paroi du canal fluidique (1).

15. Cellule de mesure selon l'une quelconque des revendications 6 à 14, **caractérisée en ce qu'**elle comporte un dispositif de collecte (18) de fluide corporel.

16. Cellule de mesure selon l'une quelconque des revendications 6 à 15, **caractérisée en ce qu'**elle comporte une zone supplémentaire (Z₄) adjacente à la première zone (Z₁), située entre l'entrée (2) et la première zone (Z₁), et/ou une zone supplémentaire (Z₅) adjacente à la seconde zone (Z₂) et située entre la seconde zone (Z₂) et la sortie (3), chaque zone supplémentaire (Z₄, Z₅) ayant une section interne réduite, inférieure aux sections internes respectives desdites première et seconde zones (Z₁, Z₂).

## Patentansprüche

1. Verfahren zum Messen der Gesamtionenkonzentration in einer Körperflüssigkeit,
**dadurch gekennzeichnet,**
**dass** es folgende Schritte enthält:
- Anlegen einer stabilen Gleichspannung zwischen einer ersten und einer zweiten Elektrode (5, 6) einer Messzelle zum Messen der Gesamtionenkonzentration in einer Körperflüssigkeit dergestalt, dass das Auftraten von Reaktionen elektrochemischer Hydrolyse des Wassers in der Körperflüssigkeit im Bereich der genannten ersten und zweiten Elektrode (5, 6) bewirkt wird,
- Messung eines Hydrolyse-Stroms zwischen den beiden Elektroden (5, 6), wobei dieser Hydrolyse-Strom durch die genannten Realctionen elektrochemischer Hydrolyse des Wassers erzeugt wird, und
- Bestimmung der Gesamtionenkonzentration in der Körperflüssigkeit durch Vergleich mit einer vorher bestimmten Eichkurve,
**dadurch, dass** die genannte Messzelle einen Flüssigkeitskanal (1) aufweist, der mindestens einen Durchgang (4) enthält, durch den die Körperflüssigkeit von einem Einlass (2) zu einem Auslass (3) strömen kann, wobei dieser Flüssigkeitskanal (1) zwischen der ersten und der zweiten Elektrode (5, 6), welche an diesem Durchgang (4) angeordnet sind, einen Innenquerschnitt aufweist, der kleiner oder gleich 1,5 mm ist, so dass die Ionenkonzentration in der Körperflüssigkeit der Faktor ist, der die Kinetik dieser Reaktionen elektrochemischer Hydrolyse des Wassers begrenzt,
und dadurch, dass die Messzelle einen Spannungsgenerator (7) und einen elektronische Mess-Schaltkreis (8) engt, die in Reihenschaltung mit der ersten und der zweiten Elektrode (5, 6) verbunden sind.

2. Verfahren nach Anspruch 1,
dadurch **gekenntzeichnet,**
dass die Messung eines Hydrolyse-Stroms durch Zeitstrommessung während des Anlegens der Gleichspannung erfolgt.

3. Verfahren nach einem der Ansprüche 1 und 2,
**dadurch gekennzeichnet,**
**dass** die Messzelle eine Verengung (9) des Innenquerschnitts des Flüssigkeitskanals zwischen der ersten und der zweiten Elektrode (5, 6) aufweist.

4. Verfahren nach einem der Ansprüche 1 bis 3,
**dadurch gekennzeichnet,**
**dass** die angelegte Spannung zwischen 2 V und 10 V, vorzugsweise zwischen 3 V und 5 V beträgt.

5. Verfahren nach einem der Ansprüche 1 bis 4,
**dadurch gekennzeichnet,**
**dass** es vor Anlegen der Spannung einen Schritt des Eintauchens der Messzelle in der Körperflüssigkeit umfasst.

6. Messzelle zum Messen der Gesamtionenkonzentration in einer Körperflüssigkeit, welche enthält:
- einen Flüssigkeitskanal (1) mit einem Einlass (2) und einem Auslass (3) für die Körperflüssigkeit,
wobei dieser Flüssigkeitskanal (1) eine erste und eine zweite Zone (Z₁, Z₂) mit jeweils einem bestimmten Innenquerschnitt sowie eine dritte Zone (Z₃), die sich zwischen der ersten Zone (Z₁) und der zweite Zone (Z₂) befindet und an diese erste und zweite Zone (Z₁, Z₂) angrenzt, enthalt, die mindestens einen Durchgang (4) für die Körperflüssigkeit aufweisen, wobei diese dritte Zone (Z₃) einen verringerten Innenquerschnitt hat, der geringer ist als die jeweiligen Innenquerschnitte der ersten und eine zweiten Zone (Z₁, Z₂), um den Ionentransport in der Körperflüssigkeit zwischen der ersten und der zweiten Elektrode (5, 6) zu begrenzern, wobei der Innenquerschnitt der dritten Zone (Z₃) kleiner als oder gleich 1,5 mm ist,
wobei diese erste und zweite Elektrode (5,6) an diesem Durchgang (4) angeordnet sind und sich im Bereich der ersten bzw. zweiten Zone (Z₁, Z₂) befinden,
- einen Spannungsgenerator (7) und einen elektronischen Mess-Schaltkreis (8), die in Reihenschaltung mit der ersten und der zweiten Elektrode (5,6) verbunden sind, wobei der Spannungsgenerator (7) ein Generator stabiler Gleichspannung ist, wobei dieser Generator dazu konfiguriert ist, eine Potentialdifferenz zwischen den beiden Elektroden (5, 6) anzulegen, um die Hydrolyse des Wassers in der Körperflüssigkeit im Bereich der genannten ersten und zweiten Elektrode (5, 6) zu bewirken,
wobei der elektronische Mess-Schaltkreis dazu konfiguriert ist, den Hydrolyse-Strom zwischen den beiden Elektroden (5, 6) zu messen.

7. Messzelle nach Anspruch 6,
**dadurch gekennzeichnet,**
**dass** der Flüssigkeitskanal (1) durch eine Abdeckkappe (11) und durch mindestens einen Teil eines Halters (12) umgrenzt ist, wobei diese Kappe (11) auf dem Halter (12) angeordnet ist.

8. Messzelle nach Anspruch 7,
**dadurch gekennzeichnet,**
**dass** der Halter (12) Drainage-Mittel (16) zum Ableiten der Körperflüssigkeit zu dem Flüssigkeitskanal (1) hin aufweist.

9. Messzelle nach einem der Ansprüche 7 und 8,
**dadurch gekennzeichnet,**
**dass** die Kappe (11) mindestens eine Entleerungsöffnung (17) aufweist, die durch die Wand der Kappe (11) verläufe und sich außerhalb der dritten Zone (Z₃) befindet.

10. Messzelle nach einem der Ansprüche 7 bis 9,
**dadurch gekennzeichnet,**
**dass** sie eine Sammelvorrichtung (18) für Körperflüssigkeit enthält und dass der Halter (12) des Flüssigkeitskanals (1) mindestens einen Teil dieser Sammel Vorrichtung (18) bildet.

11. Messzelle nach einem der Ansprüche 6 bis 10,
dadurch **gekenntzeichnet,**
**dass** der Flüssigkeitskanal (1) mindestens eine Verengung (9) im Bereich der dritten Zone (Z₃) aufweist, die den Durchgang (4) enger macht.

12. Messzelle nach einem der Ansprüche 6 bis 11,
**dadurch gekennzeichnet,**
**dass** die erste oder zweite Elektrode (5, 6) jeweils von einem Draht gebildet werden, der durch den Flussigkeitskanal (1) hindurch verläuft.

13. Messzelle nach einem der Ansprüche 6 bis 11,
**dadurch gekennzeichnet,**
**dass** die erste und zweite Elektrode (5, 6) in der Wand des Flüssigkeitskanals (1) gebildet sind.

14. Messzelle nach einem der Ansprüche 6 bis 11,
**dadurch gekennzeichnet,**
**dass** die erste und zweite Elektrode (5, 6) an einer und der gleichen Wand des Flüssigkeitskanals (1) angebracht sind.

15. Messzelle nach einem der Ansprüche 6 bis 14,
**dadurch gekennzeichnet,**
**dass** sie eine Sammelvorrichtung (18) für Körperflüssigkeit enthalt.

16. Messzelle nach einem der Ansprüche 6 bis 15,
**dadurch gekennzeichnet,**
**dass** sie eine zusätzliche Zone (Z₄) aufweist, die an die erste Zone (Z₁) angrenzt und sich zwischen dem Einlass (2) und der ersten Zone (Z₁) befindet, und/oder eine zusätzliche Zone (Z₅) aufweist, die an die zweite Zone (Z₂) angrenzt und sich zwischen der zweiten Zone (Z₂) und dem Auslass (3) befindet, wobei jede zusätzliche Zone (Z₄, Z₅) einen verringerten Innenquerschnitt hat, der geringer ist als die jeweiligen Innenquerschnitte der ersten und der zweiten Zone (Z₁, Z₂).

## Claims

1. Method for measuring the global ion concentration of a body fluid, **characterized in that** it comprises the following steps;
- application of a stable DC voltage between first and second electrodes (5, 6) of a cell for measuring the global ion concentration of a body fluid so as to cause electrochemical hydrolysis reactions of the body fluid water to occur at the level of said first and second electrodes (5, 6),
- measurement of a hydrolysis current between the two electrodes (5, 6), said hydrolysis current being generated by said electrochemical hydrolysis reactions of water and,
- determination of the global ion concentration of the body fluid by comparison with a previously defined calibration curve,
**in that** the measuring cell comprises a fluid channel (1) arranging at least one passage (4) enabling the body fluid to flow from an inlet (2) to an outlet (3), said fluid channel (1) having an internal cross-section less than or equal to 1.5 mm between the first and second electrodes (5, 6) arranged on said passage, so that the ion concentration of the body fluid is the factor limiting the kinetics of said electrochemical hydrolysis reactions of water,
and **in that** the measuring cell comprises a voltage generator (7) and an electronic measurement circuit (8) connected in series to the first and second electrodes (5, 6).

2. Method according to claim 1, **characterized in that** measurement of the hydrolysis current is performed by chronoamperometry during application of the DC voltage.

3. Method according to one of claims 1 and 2, **characterized in that** the measuring cell comprises a constriction (9) of the internal cross-section of the fluid channel between the first and second electrodes (5, 6).

4. Method according to any one of claims 1 to 3, **characterized in that** the voltage applied is comprised between 2V and 10V, preferably between 3V and 5V.

5. Method according to any one of claims 1 to 4, **characterized in that** it comprises an immersion step of the measuring cell in the body fluid before the voltage is applied.

6. Cell for measuring the global ion concentration of a body fluid which comprises:
- a fluid channel (1) with an inlet (2) and an outlet (3) of the body fluid, and said fluid channel (1) comprising first and second zones (Z₁, Z₂) each having a defined internal cross-section and a third zone (Z₃) situated between the first zone (Z₁) and the second zone (Z₂) and adjacent to said first and second zones (Z₁, Z₂) and arranging at least one passage (4) for the body fluid,
said third zone (Z₃) having a reduced internal cross-section, smaller than the respective internal cross-sections of said first and second zones (Z₁, Z₂), to limit ionic migration between first and second electrodes (5, 6) in the body fluid, the internal cross-section of the third zone (Z₃) being less than or equal to 1.5 mm,
said first and second electrodes (5, 6) being arranged on said passage (4) and being respectively situated at the level of the first and second zones (Z₁, Z₂),
- a voltage generator (7) and an electronic measurement circuit (8) connected in series to the first and second electrodes (5, 6),
the voltage generator (8) being a generator of stable DC voltage, said generator being configured so as to apply a voltage difference between the two electrodes (5, 6) to generate electrochemical hydrolysis of the body fluid water at the level of said first and second electrodes (5, 6),
the electronic measurement circuit being configured to measure the hydrolysis current between the two electrodes (5, 6).

7. Measuring cell according to claim 6, **characterized in that** the fluid channel (1) is delineated by a cover (11) and by at least a part of a support (12), said cover (11) being arranged on the support (12).

8. Measuring cell according to claim 7, **characterized in that** the support (12) comprises means for draining (16) the body fluid to the fluid channel (1).

9. Measuring cell according to one of claims 7 or 8, **characterized in that** the cover (11) comprises at least one drain hole (17) passing through the wall of the cover (11) and situated outside the third zone (Z₃).

10. Measuring cell according to any one of claims 7 to 9, **characterized in that** it comprises a body fluid collecting device (18) and **characterized in that** the support (12) of fluid channel (1) forms at least a part of said collecting device (18).

11. Measuring cell according to any one of claims 6 to 10, **characterized in that** the fluid channel (1) comprises at least one constriction (9) at the level of the third zone (Z₃) making the passage (4) narrower.

12. Measuring cell according to any one of claims 6 to 11, **characterized in that** the first or second electrodes (5, 6) are both formed by a wire passing through the fluid channel (1).

13. Measuring cell according to any one of claims 6 to 11, **characterized in that** the first and second electrodes (5, 6) are formed in the wall of the fluid channel (1).

14. Measuring cell according to any one of claims 6 to 11, **characterized in that** the first and second electrodes (5, 6) are deposited on one and the same wall of the fluid channel (1).

15. Measuring cell according to any one of claims 6 to 14, **characterized in that** it comprises a body fluid collecting device (18).

16. measuring cell according to any one of claims 6 to 15, **characterized in that** it comprises an additional zone (Z₄) adjacent to the first zone (Z₁), situated between the inlet (2) and the first zone (Z₁), and/or an additional zone (Z₅) adjacent to the second zone (Z₂) and situated between the second zone (Z₂) and the outlet (3), each additional zone (Z₄, Z₅) having a reduced internal cross-section, smaller than the respective internal cross-sections of said first and second zones (Z₁, Z₂).
